# EUROPEAN PATENT SPECIFICATION

(11) **EP 2 023 994 B1**
(45) Date of publication and mention of the grant of the patent: **29.05.2013**
(21) Application number: 07725247.6
(22) Date of filing: 15.05.2007
(51) Int. Cl.: A61M 25/00, A61M 25/06, A61B 5/00, A61M 25/01

(54) **CATHETER HAVING AN OBLONG SLIT**
KATHETER MIT LÄNGLICHEM SCHLITZ
CATHÉTER À FENTE DE FORME OBLONGUE

(30) Priority: 15.05.2006 EP 06009967; 15.05.2006 US 800540 P
(43) Date of publication of application: 18.02.2009
(73) Proprietor: Joanneum Research Forschungsgesellschaft mbH, 8010 Graz (AT)
(72) Inventor: BODENLENZ, Manfred, A-8010 Graz (AT); SCHAUPP, Lukas, A-8010 Graz (AT)
(74) Representative: Dilg, Haeusler, Schindelmann Patentanwaltsgesellschaft mbH
(86) International application number: PCT/EP2007/004330
(87) International publication number: WO 2007/131780

(56) References cited:
- EP-A- 1 212 978
- EP-A1- 1 177 759
- WO-A-99/48550
- WO-A2-03/086209
- DE-A1- 19 612 105
- US-A- 4 801 297
- US-A1- 2003 167 031
- US-A1- 2004 168 934

## Description

The invention relates to a catheter system as defined in claim 1. The invention further relates to a device.

Moreover, the invention relates to a method of manufacturing a catheter.

Beyond this, a medical method is described.

Furthermore, a method of using a catheter for measuring at least one physiological parameter in a physiological object is described.

Microdialysis is a known technique to continuously sample substances in tissue. For this purpose, semi-permeable membranes are used to access substances in tissue.

WO 88/05643 A1 discloses that, in order to determine at least one parameter of interest in a living organism, a perfusion fluid is directly introduced in the tissues. After its partial balancing by the tissue parameter of interest, the perfusion fluid is collected and analyzed for the parameter of interest, as well as for endogenous or exogenous marker properties indicative of the degree of interaction between the perfusion fluid and the tissue, in such a way that the parameter of interest can be determined with the help of such characteristic properties. This process creates in the tissues, i.e. in the closed cellular structure, a previously inexistent cavity, in which the perfusion fluid introduced in the tissue interacts directly with the organic tissue, with no intervening membranes.

WO 88/05643 A1 does not use a membrane, but accesses substances in tissue using circular macroscopic openings and septa in a catheter.

However, it may be costly and time-consuming to manufacture the device of WO 88/05643 A1.

WO 99/48550 A discloses an endocardial catheter including a plurality of longitudinally extending openings adjacent intermediate portions at its distal end. The catheter is acutable from a retracted or collapsed mode, wherein the sealed openings are arranged around the tubular catheter surface to an expanded mode. The plurality of longitudinal openings in the catheter wall enable radial expansion of the tubular surface at the distal end, so that intermediate portions of the tubular catheter surface are moved to an operative position radially outward from their position in the retracted mode. In the expanded position, the intermediate portions form wings around the distal end, revealing a cavity within the tubular catheter for the release of contrast material or other fluid into endocardial sites through the longitudinal openings.

US 5,702,365 discloses a dual-lumen blood-treatment catheter having inner and outer lumina open towards the patient end. The catheter has expandable portions in the outer lumen located near the patient end for atraumatically preventing collapse of the blood vessel to ensure free flow of blood into and out of the catheter. The outer lumen may have a plurality of slits around its circumference which form slats therebetween. Withdrawal of the inner lumen relative to the outer lumen causes the slits to open, thereby bowing the slats to expand the outer lumen.

EP 0,248,670 discloses an apparatus and a method of use for removing specified dialyzable components from a complex reference fluid such as, for example, blood. A semi-permeable tubular dialysis membrane is provided which is inserted into the reference fluid. An equilibration fluid is injected into the tubular membrane and allowed to equilibrate with the outside reference fluid. The equilibrated fluid is then removed for analysis, or possibly analyzed in situ.

WO 03/086209 discloses an angioplasty device and particle trap for use in removal of a particle from a small diameter or vessel-like structure. A catheter for insertion into a vessel-like structure may be provided, the catheter having a catheter wall and a movable member, a trap operably connected to the catheter wall and to the movable member, wherein relative motion between the catheter wall and the movable member actuates the trap. The expanded trap may be formed from struts in a spiral-shaped configuration. The contracted trap may form a waist to create a pinch-point to trap particles. The contracted trap may form a cocoon-like structure to further trap particles. The angioplasty device may include a handle to actuate the trap from a contracted position to an expanded position and return to a contracted position. The handle provides rotational or longitudinal or both types of movement to actuate the trap.

Document US 2003/0167031 A1_discloses a system and a method for site specific therapy comprising a catheter apparatus, wherein the catheter apparatus includes one or more semipermeable microcatheters comprising a semipermeable membrane for performing microdialysis.

Document WO 99/48550 discloses a catheter for media injection, in particular for the release of contrast material into endocardial sites through longitudinal openings.

It is an object of the invention to provide an efficient catheter system as defined in claim 1.

In order to achieve the object defined above, a microperfusion catheter system, a device, a method of manufacturing a microperfusion catheter system according to the independent claims are provided.

According to an exemplary embodiment of the invention, a microperfusion catheter system for insertion into tissue of a physiological object is provided, the microperfusion catheter system comprising a tube or structure made of a flexible biocompatible impermeable material, the structure having a lumen, a first end portion and a second end portion, and an oblong slit (or an oblong hole or an oblong recess) formed in a wall of the tube (preferably extending along a longitudinal axis of the tube, whereas alternative orientations and alignments of the oblong slit are possible). The microperfusion catheter system further comprises a delivery unit for delivery of perfusion fluid to the lumen of the structure in a manner to allow for an exchange of substances between the tissue and the perfusion fluid via the oblong slit, wherein the delivery unit is connected to the first end portion of the structure for delivering the perfusion fluid to the lumen of the structure, wherein a drain unit is connectable to the second end portion of the structure for draining the perfusion fluid after the exchange of substances between the tissue and the perfusion fluid via the oblong slit. The microperfusion catheter system comprises a delivery unit for delivery (or supply) of perfusion fluid to a lumen of the structure in a manner to allow for an exchange (monodirectionally or bidirectionally) of substances between the tissue and the perfusion fluid (that is from the tissue to the perfusion fluid, and/or in the opposite direction) via the oblong slit.

According to another exemplary embodiment of the invention, a medical device is provided comprising a microperfusion catheter system having the above-mentioned features, and an insertion element (particularly an insertion needle) to be connected to the catheter for facilitating insertion of the catheter into the tissue of the physiological object.

According to still another exemplary embodiment of the invention, a method of manufacturing a microperfusion catheter system for insertion into tissue of a physiological object is provided, the method comprising the steps of providing a tube or structure made of a flexible biocompatible impermeable material, the structure having a lumen, a first end portion and a second end portion, and forming an oblong slit in a wall of the tube extending along a longitudinal axis of the tube. Further, a delivery unit is formed for delivery of perfusion fluid to a lumen of the structure in a manner to allow for an exchange of substances between the tissue and the perfusion fluid via the oblong slit, wherein the delivery unit is connected to the first end portion of the structure for delivering the perfusion fluid to the lumen of the structure, wherein a drain unit is connectable to the second end portion of the structure for draining the perfusion fluid after the exchange of substances between the tissue and the perfusion fluid via the oblong slit.

According to another example, a catheter having the above-mentioned features is used for measuring at least one physiological parameter in tissue of a physiological object.

The term "oblong slit formed in a wall" may include embodiments in which the slit is a through hole entirely penetrating the wall and may include embodiments in which the slit is a blind hole in the wall without entirely penetrating the wall. The slit may be an indentation or a recess of removed wall material which removal locally thins the wall compared to surrounding wall portions. It may be a groove in the wall as well. Locally thinning a wall may be realized as well using a cutting technique or the like.

The term "physiological object" may particularly denote any human being, any animal, and any plant (any organism).

The term "impermeable" may particularly denote a material property of the tube, namely that the tube cannot be traversed - in any significant manner or quantity - by fluidic or solid particles. In contrast to this, a dialysis membrane is permeable for substances being smaller than a cut-off size of the membrane.

The term "flexible" may particularly denote a material property of the tube, namely that the tube can be reversibly deformed under the influence of an external force.

The term "biocompatible" may particularly denote a material property of the tube, namely that the tube, when inserted in living tissue, does not harm or negatively influence the physiological conditions at such a location in a body.

The term "physiological parameter" may particularly denote any parameter which is related to the physiology of a living organism, for instance the metabolism, etc. Such a physiological parameter may include the concentration of an exogenous or endogenous marker, a protein concentration, etc.

The term "physiologically active substance" may particularly denote any substance which may have an effect on the physiology of the living organism, for instance a medication, a drug, etc.

The term "structure" may denote any piece of material based on which a catheter may be built. It may be a planar structure, a three-dimensional structure, etc. Examples are tubes, circles, polygons.

The term "physiologically inert substance" may particularly denote any substance which may be free of causing any effect on the physiology of a living organism, for instance mannitol, inulin, etc. Mannitol is a sugar-like substance which is essentially not metabolized by the human body and thus remains in the body with an essentially constant concentration in different regions of the body for a significant time.

The term "perfusion fluid" may particularly denote a fluid (such as a buffer, water, a medication, etc.) which may be brought in interaction with a body fluid/fluidic sample/tissue via the slitted structure so that a material transport from the body fluid/fluidic sample/tissue to the perfusion fluid (or vice versa) may allow to analyze a component of the body fluid/fluidic sample/tissue by analyzing the perfusate. The term "perfusion fluid" may denote the liquid entering and leaving a lumen of the catheter, respectively.

The catheter is be manufactured from any impermeable material. However, is an alternative example not forming part of the invention, the catheter may be made of a permeable membrane/material allowing exchange of substances via the one or more oblong hole(s) and via the permeable membrane!

The shape of the catheter may be tubular. However, other shapes are possible as well, for instance a (rectangular) stripe-like geometry with an oblong hole along one of the axes which define the rectangle.

The catheter may be used, for example, to assist or simplify insertion of one or more sensors into a physiological object. Such a sensor may be shifted along a lumen of the catheter. When being positioned within the physiological object, the sensor may be functionally coupled (for instance for fluid communication) with material of the physiological object via the oblong slit(s).

It is possible to implant the catheter into a physiological object and to extract body substances (for instance a body fluid like an interstitial fluid) from the physiological object through the catheter to an exterior of the physiological object. For instance, fluid may be sucked out of the body by applying a negative pressure. Or fluid may be actively (pump) or passively delivered to the catheter, and the (partially equilibrated) effluent may be conducted out of the catheter actively or passively. Such an extracted body fluid may be supplied to a sensor for further analysis, and/or may be collected in one or more fractions.

The fluid can be collected from the catheter by pure sucking (applying negative pressure), or by active (pump)/passive delivery of perfusion fluid to the catheter and active/passive collection of fluid from the catheter. This may be helpful for sampling of substances and delivery of substances.

The oblong slit(s) formed in the material may be straight (for instance a straight line).

Due to the open surface of the catheter (in case of no membrane, and in case of an open slit) also large substances, bound substances, lipophilic substances can be collected or delivered.

According to an exemplary embodiment of the invention, a catheter may be provided which may allow for an equilibration of fluid concentration differences within and without the catheter tube through one or more oblong holes formed in a wall of the tube. By providing oblong holes having a length which is essentially larger than the width, the tube may be perforated in a longitudinal direction so as to improve flexibility of the entire system. The tube itself may be made of an essentially flexible material allowing the tube to snugly fit to adjacent tissue and to be inserted into a physiological object, e.g. the human body. This flexibility may be further improved by forming one or more longitudinal slits in a surface of the tube so as to enable fluid communication between an interior and an exterior of the tube via the oblong slit. Since, for manufacturing the catheter, it is sufficient to simply cut an oblong hole in a, for instance, cylindrical tube. Such a manufacture is fast, easy, may be defined and adjusted by a user, and may be performed with very simple tools like a knife or a scalpel. Via a delivery unit, a perfusion fluid may be supplied to a lumen of the slitted tube to enable an exchange of substances between an outer portion of the tube and an inner portion of the tube via the one or more slits. This may allow to sample a fluid by an equilibration of concentrations exterior and interior of the lumen via the slits. However, the width of the slits should be sufficiently small to prevent a majority of the perfusion fluid (acting as a probe) from leaving the lumen. For the purpose of sampling a test fluid, a perfusion fluid enters a first end portion of the tubular slitted catheter, streams through the lumen, exchanges substances with the test fluid through diffusion-like processes through the slit(s), and leaves a second end portion of the tubular slitted catheter. The draining fluid may or may not be analyzed. The fluid exchange through the slit(s) may or may not introduce a drug into the surrounding fluid. The exchange may include a simultaneous flow of substances into the lumen and out of the lumen.

According to an exemplary embodiment, the catheter may be inserted into tissues or unit cell structures ("Zellverband") of an organism, rather than into fluid-filled cavities of an organism.

The catheter may feature lumina allowing simultaneous perfusion of the tissue with a perfusion fluid (i.e. inflow) and withdrawal (i.e. outflow) of perfusion fluid. One lumen may be used for the (continuous) inflow of a perfusion fluid, whereas the other lumen may be used for the (continuous) outflow of the partially equilibrated perfusion fluid that carries information/substances from the organism. Inflow and outflow lumen may be arranged in such a way relative to the catheter's section with openings that the perfusion fluid is forced to pass the openings. Thus, a high or maximum degree of exchange or interaction between perfusion fluid and ambience/environment may be assured.

Such a arrangement can be achieved easily by a linear catheter design (inflow lumen - segment with open surface - outflow lumen) as may be suitable for intradermal applications. Also a concentric design is feasible requiring the smaller lumen located within the larger lumen for inflow and outflow or vice versa. Alternatively, a multi-lumen tubing can be used.

A catheter according to an exemplary embodiment may serve for a simultaneous supply and drain of perfusion fluid for a (continuous) perfusion of the tissue.

According to an exemplary embodiment, a single-component single-material integrally formed catheter manufactured on the basis of a tubular film may be provided to serve as a catheter having an oblong opening or recess in the catheter wall. For instance, such an oblong hole may have dimensions of 20mm in length and 0.2mm in width and may extend along a longitudinal axis of the tube. Providing one or more of such cuts along the circumference of the tube may allow a high degree of mechanical flexibility when inserting such a tube into tissue of a human or animal body, thus effecting the surrounding tissue only in a marginal manner. This may allow the tube to remain within the body for several days and more, since the flexible catheter is capable of supporting, assisting, following or allowing a deformation of the tissue (for instance when a person moves her or his body).

Therefore, in contrast to conventionally used stiff geometries, embodiments of the invention allow to obtain a high degree of flexibility in combination with an efficient fluid communication feature ensured by the oblong hole(s). No restrictions with respect to sizes of particles to be exchanged through the oblong hole have to be considered, contrary to catheters made of a permeable material.

The single-component catheter according to an exemplary embodiment may be free of connecting elements or attaching elements and may be free of adhering connections as well. Optionally, the flexible tube having the longitudinal oblong slits may be twisted for insertion into the body so as to reduce the exterior diameter for inserting the catheter in tissue and at the same time to widely close the slits during the insertion procedure. For the application in relatively though or sensitive tissues (e.g. dermis of the skin), both the smaller diameter and the closed surface are beneficial. Less force is needed for the insertion, and this in combination with the smoother catheter surface less stresses and harms the adjacent tissue, such that subsequent measurements better mirror a physiological situation. For the application in very soft tissues (e.g. subcutaneous adipose tissue) the widely closed surface prevents excessive intrusion of tissue material into the catheter structures during insertion.

According to an exemplary embodiment, a catheter device for accessing tissue fluid or interstitial fluid in living organisms may be provided. Such a catheter may be particularly suitable for insertion into the skin (dermal application) for the continuous accumulation and continuous or non continuous withdrawal of the (accumulated) fluid) of and substances contained therein.

On the basis of a commercially available biocompatible tubular material, it is possible to form, using very simple cut/shape techniques in a purely manual manner a minimal complex, highly flexible single component and therefore very secure and cost-efficient catheter, which may be particularly compatible with requirements for application in the skin of a human being.

As alternatives to such a cutting technique, the catheter may be manufactured using procedures like laser processing, blanking, drilling and molding. The catheter may be formed from a raw profile material or from fluidic plastics. However, it should be ensured that, as a consequence of the processing procedure, the flexibility of the catheter may be maintained or even emphasized and that the tensile strength remains high (as high as when manufactured by manual longitudinal cutting). The manually made catheter features a particularly high tensile strength (important for safe introduction into/removal from skin), because longitudinal cutting does not cut/interrupt internal longitudinal structures..

According to an exemplary embodiment of the invention, it may be possible to avoid a membrane to allow a free exchange of substances between perfusion fluid and surrounding tissue. A (conventional dialysis) membrane does not allow such a free exchange of all molecules. In contrast to this, the material of the tube may be formed of an impermeable material so that any exchange of substances is enabled in a spatially defined manner only through the oblong hole(s). Therefore, no requirements with respect to substance dimensions and properties have to be considered with the catheter according to an exemplary embodiment of the invention. Furthermore, the structure remaining in the tissue is highly flexible and is able to follow deformations in the tissue. Consequently, once the catheter is inserted into a tissue, its exact geometrical appearance, its exchange surface(s) and the displaced tissue volume is not known, which is in contrast to state of the art catheters. Thus, the catheter function remains intact even in the presence of such an external deformation or force.

According to an exemplary embodiment, a single component catheter (that is to say a catheter which is consecutively made of a homogeneous material) may be manufactured from a single piece of a substrate (for instance a tubular substrate). Since a single component/a single part may be sufficient, no connection/adhering between parts of the catheter is necessary for obtaining an exchange surface between tissue and perfusion fluid which passes its way along the longitudinal structures of the catheters. The structures may look like a random shaped bundle of threads, and an internal space (interior/cavity) of the catheter may either not exist, or have a previously unknown shape.).

The catheter is manufacturable in a purely manual manner with very simple tools, like a cutting knife. The catheter is easy in manufacture and secure in use. There is essentially no risk caused by chipping, particles, ridges, or undesired material influences which may be caused by conventional methods for manufacture of catheters.

Catheters according to exemplary embodiment may have a high resistance against tearing or breaking, since a longitudinally oriented (fibre) structure of the starting material of the material may be maintained. The flexibility of the catheter may further promote the possibility of introducing the catheter in all kinds of tissue using a suitable implantation technique.

The small diameters and the miniature dimensions of the catheter allow to carry out tissue layer specific measurements (for instance in the dermis of the skin, 'intradermal', 'intracutaneous'). Therefore, the catheter according to an exemplary embodiment of the invention may be appropriate as a cutaneous or subcutaneous probe for locally monitoring effects of physiologically active substances (like medications or cosmetics) applied from outside of the skin or administered in another manner.

The catheter structure according to an exemplary embodiment may allow even problematic target substances a minimum of surface for adsorption, which makes unique measurements possible.

In contrast to conventional microdialysis systems, embodiments of the invention are appropriate for a high number of applications (in contrast to a membrane excluding many substances for a measurement or adsorbing of a substance to be examined), the macroscopic oblong hole of a catheter according to an exemplary embodiment is appropriate for many measurement principles.

An exemplary field of application of such a catheter is an online lactate monitoring of a human body using a portable device, for instance in the field of sports.

Catheters according to exemplary embodiments of the invention have been successfully tested in animal and human experiments. A dermatological analysis of the results has been successful.

With respect to the sampling of small/hydrophilic substances, catheters according to exemplary embodiment show a remarkable sampling efficiency, and a good suitability for online monitoring of the metabolism (for instance with regard to glucose or a lactate levels).

With respect to the sampling of large/lipophilic substances, large molecular inflammation parameters (cytokines) may be recovered from the tissues. It is also possible, using catheters according to an exemplary embodiment, that is, using a minimally invasive examination method, to collect and investigate superlipophilic drugs (medication) in tissues.

The possibility of the simultaneous examination of the concentration of such substances in tissue being subject of a therapy ("pharmacokinetics") with the effect obtained in the tissue (for instance via cytokines, "pharmacodynamic") may allow the catheter to be used as a support for the development of new medications. In pharmaceutical industry, when developing substances for instance with respect to skin diseases (dermatitis, psoriasis, etc.), the catheter may be used for a minimal invasive investigation for a direct proof of the effect in human experiments.

An exemplary field of application of embodiments of the invention are the investigation of medication concentrations and their local effect in tissue, particularly in the skin (dermatology and all dermatologic products).

According to an exemplary embodiment, the manufacture of catheters for the access to tissue fluid in living organisms may be provided. The mentioned catheters may be suitable to be used to collect/sample 'tissue fluid' (which may also be denoted as 'interstitial fluid' or 'extracellular fluid') from living tissues, or at least the substances/drugs therein.

It is possible to use, as a material for the longitudinal slit including catheter (alternatively to an impermeable tube material), a membrane material allowing a diffusion through the membrane in addition to a material exchange through the oblong slit.

For realizing such a catheter, particularly for enabling access to a tissue fluid in living organisms, such a catheter may be manufactured using pure manual manufacture procedures based on a continuous piece of a biocompatible tubular material. With such a manufacturing procedure and catheter shape, it may be dispensable to attach complexly shaped parts or materials and to implement high tech processing methods. The low complexity of the catheter according to an exemplary embodiment with respect to shaping and material may allow a very easy and cost efficient manufacture. This simplicity of the catheter constitution as well as the manufacturing method may further ensure a high security when employing such a catheter in living organisms.

Catheters according to exemplary embodiment of the invention may be manufactured purely manually, that is to say using very simple tools. The starting material and/or the starting form (for instance tubular material) is standard and is commercially available with low cost. All catheter segments (parts) remaining in the organism, and the conduits can be manufactured (including the connection conduits) from a single continuous piece of such a standard raw material, and the manufacture of connections between components/materials, for instance using adherence techniques, may be dispensable.

Catheters according to exemplary embodiments of the invention can be manufactured with very small outer dimensions (diameter), for instance in the order of magnitude of centimeters to millimeters and below. While maintaining a high degree of flexibility, and while enabling very small diameters, a high resistance to tearing or breaking may be obtained, making the catheters suitable for use within the living body, for instance in layers of the skin.

The insertion of the catheter can be performed in a gentle manner to reduce the harm to the tissue. When using a helical structure, pulling and twisting the helical structure during implantation may allow to reduce the catheter diameter so that the helical opening of the helically twisted oblong hole is temporarily closed completely. A closed surface and a reduced diameter may reduce friction and deterioration of the tissue to be examined, by the implantation procedure.

The manner of manufacture and the single component construction of this catheter may result in an improved security in application. The danger of ripping off or breaking off of catheter components due to manufacturing or material problems and the danger that a part of the catheter remains in the tissue is reduced.

The membrane-less exchange surface of the catheter, when implanted in the tissue, locally allows the determination of all substances present in the tissue fluid. It may be prevented or suppressed that substances are excluded as would be the case by membranes due to cutoffs or membrane adsorption. As a consequence of this very efficient exchange via the catheter, it is possible to examine even larger molecular or lipophilic substances without a specific addition to the perfusion fluid. Since the catheters according to exemplary embodiment of the invention are particularly suitable for being used in the skin, they enable the examination of (even larger molecular) body own substances, which may play a role in the skin in the context of many skin diseases (for instance cytokines). Since the catheters are permeable also for immunomodulating substances (for instance highly lipophilic medication molecules), which may have an influence on such skin diseases, may make it possible to investigate the local medication concentration and the medication effect in combination.

Thus, the catheter according to an exemplary embodiment may be used for clinical studies for the fast retrieval of an advantageous or optimum medication composition. In such a scenario, the catheter may be used for the measurement of a medication concentration in the tissue for the direct and/or indirect proof of the medication effect, namely that substances are released or not released. Furthermore, embodiments of the invention may be implemented in medical science for physiological fundamental research, and also in the context of the (for instance continuous) monitoring (for instance glucose monitoring, lactate monitoring) of one or more physiological parameters.

Catheters according to exemplary embodiments of the invention may be used in all areas of life science, for instance for research on living organisms, studies in the context of official approval of medications, etc.

In pharmaceutical industry, it is necessary to make a decision between a plurality of potentially efficient substances and formulations when developing a new medication. This may involve a time consuming and cost-intensive test of such medications in a physiological subject. For many substances/formulations, no examination methods are available which are gentle for the experiment participant and which deliver the required data for allowing a meaningful pre-selection among substances and formulations in an early test phase. An appropriate procedure using a catheter according to an exemplary embodiment of the invention may accelerate the medication development and may reduce the costs significantly. Particularly in the field of dermatology, many products are developed, for instance for curing skin diseases, health cosmetics, for treating sunburn, etc.

Furthermore, embodiments of the invention may be implemented in the context of continuous glucose monitoring.

Catheters according to exemplary embodiments are well suited in clinical research areas for the sampling or administering of large molecular substances (for instance peptides or peptide hormones). Thus, it may be made possible to get access to tissue and tissue fluid in living organisms.

According to an exemplary embodiment, a catheter may be provided as a single component device including all functionally relevant structures which may be obtained using a very simple manufacturing procedure. Such a catheter may be manufactured from a single continuous piece of a commercially available and biocompatible tubular material. A piece of such a biocompatible medical tubular material (for instance PTFE) may first be provided with an oblong hole along a predefined length of a part of the tubular wall using a simple manual cutting technique. By providing such an oblong through hole, the interior lumen of the tube is opened along the length of such an oblong hole with respect to the environment. For the cutting process, it is possible to use a clean and sharp knife (for instance a sterile packed scalpel).

Maintenance of a specific cutting depth along the cut length may be simplified by the preceding insertion of a wire (for instance a surface hart spring steel wire) into the tube. After cutting, the tube can be twisted in such a manner that the slit-shaped opening can assume a helical form (or spiral form) along the tubular surface. This helical shaped tube can be heated temporarily (for instance using hot air) and can be maintained permanently in a helical shape by subsequent cooling of the material. Also this procedure of the conservation of the shape can be simplified using an interior positioned wire.

Located in living tissue, the uninterrupted helical opening may enable a flowing fluid to come into direct contact with the surrounding tissue/tissue fluid along a sufficiently large length, thereby allowing to exchange substances in both directions. Being situated in soft tissue (for instance fatty tissue), the remaining helical tubular wall may fulfill the function of mechanically supporting tissue parts so that the tissue cannot close the streaming channel completely. The risk of an occlusion of the drain of the helix by biological material can also be reduced when using medical yarn material (like surgical sewing material, for instance multi fibril material) in tube and helix.

When using such catheters in tense tissue (for instance skin), the addition of yarn material may be dispensable, since the probability of occlusion is usually lower. When using such a catheter in tense skin layers of animals or human beings, it may be possible to omit the twisting of the tube for generating the helical shape.

The insertion of such tubular parts and/or catheter parts in tissue may be enabled by connecting the tube with a sharp hollow needle. Even this connection can be carried out without high expenditure and without additional materials (for instance without adherence), by tapering the tubular material by pulling in an axial direction (optionally during heating the material). This may be done in such a manner that the tapered part of the tube can be slid in an edgeless end of a hollow needle and may be squeezed with it manually and mechanically. Using an attached implantation needle, the catheter can be inserted with low friction directly in the skin or through the skin in surface near tissue parts, without a need to perform a preceding perforation of the skin. Due to the fixed squeezing connection, the essentially friction-free transition between needle and catheter (tube) and the mechanical stability with respect to pull forces of the tubular material (for instance PTFE, Teflon) in axial direction, the risk of tearing off a catheter part during implanting and explanting, and thus the risk that catheter parts remain in living tissue may be kept small.

The insertion of the catheter and the removal of the catheter can be performed gently for the tissue, if desired. By selectively twisting and/or pulling the helical structure during implantation, the helical opening can be closed, and the catheter diameter is further reduced. Closed surface and reduced diameter reduce friction and deterioration of the examined tissue during implementation. An open exchange surface towards the tissue and the final structure in the tissue can then be generated after implantation by relaxing the surface parts of the catheter. The use of the method for the selective influence of the structure and the tissue from exterior may allow as well a gentle explantation of the catheter without further tissue deterioration.

When increasing the number of longitudinal openings along the exterior cross section, a number of consecutive fibre shaped highly flexible and mechanically stable longitudinal structures may be obtained which take an arbitrary position in the tissue with respect to one another, thereby forming longitudinally oriented hollow spaces through which a supplied perfusion fluid may flow. There is no need to define a 'preformed' channel/path, as considered to be necessary according to WO 88/05643 A1. Such an undefined flow path and the way of generating hollow spaces for draining the perfusion fluid may be advantageous. The displaced tissue volume may be very small, and therefore the influence on the tissue and/or the adjacent cell structures and their physiologically functions may be small as well. The exchange between perfusion fluid and the tissue may be improved or optimized, and the determined concentrations may reflect actual physiological concentrations in the extra cellular space in an advantageous manner.

In the case of motions/shifts in the tissue, the flexible catheter structure may follow tissue deformations and therefore generates only a small pressure onto surrounding structures.

For the application in various surface near tissues (for instance skin and sub-skin tissue) in clinical studies, a simple linear catheter shape may be advantageous. It can be inserted in a gentle manner in the tissue to be investigated, since such an implantation device (for instance a needle) can be of a very small diameter. In such scenarios the catheter penetrates the body surface at more than one position.

When the exchange surface of the catheter shall be implemented into deeper situated tissue parts, or when the skin shall only be penetrated at one position, an alternative implantation procedure may be applied. The catheter may be inserted along a part (for instance half of the catheter length) into a hollow injection cannula, and the remaining tubular part can be guided back or returned along its surface. When the injection cannula is inserted into the tissue and is pulled back in a gentle manner, the flexible catheter may remain with the exchange surface in the target tissue. Even for such a catheter type, additional (multi-fibril) sewing material in the interior of the catheter can avoid or suppress occlusion of the drain during operation. The cross-section of all catheter parts can be significantly reduced by pulling (for instance further simplified by heating), in order to obtain a partially decreased outer and inner diameter. The flexibility of the tapered tube material is increased, so that motions of exterior catheter parts are transferred to the implanted catheter parts only in a reduced manner, which may have a good and gentle effect onto the tissue.

A further possibility to generate a lumen for the backflow of the perfusion fluid through the same body opening is to reduce the diameter of the back-guiding tubular part so that it can be guided within the exchange area/within the helix and within the tubular lumen to be supplied back to the body surface and/or to the exterior.

Using a two-luminal or multi-luminal tube, one lumen can be used as a backflow. Such catheter shapes can be inserted very easily through the human of a hollow needle into the tissue.

Catheters of the defined type have already been tested in animal and human experiments. Such catheters were very easily insertable into the tissue, also with respect to sampling properties for research and monitoring purposes. It has been possible, using such catheters for continuous sampling, to recover a super-lipophilic medication in the human skin. Further human experiments with such a catheter type for continuous monitoring of metabolic parameters show a high reproducibility of the results. For instance, the glucose, lactate and ion data of four parallel oriented catheters in the skin and three parallel oriented catheters in abdominal subcutaneous fatty tissue indicated that such catheters allow a high degree of retrieval of substance in the catheter drain, that the catheters own a good compatibility and that they enable a very good reconstruction of the characteristics of the capillary glucose concentration.

This catheter type has also been successfully implemented in experiments in order to continuously monitor the lactate concentration during intense exercise.

According to an exemplary embodiment, a device and/or catheter for accessing tissue or tissue fluid in living organism is provided, wherein the surface of the catheter is not closed but open towards the tissue. The structures of the catheter do not form a pre-formed channel of a defined and constant dimension, but the structures may in contrast to this be configured that their shape and position as well as the tissue volume displaced by the catheter is modifiable after insertion of the catheter into the tissue.

Such a catheter may be manufactured for accessing parameters in tissue or tissue fluid in living organisms by using an uninterrupted piece of a homogeneous fluid tight tube material, wherein such a tube may be modified with very simple and purely manual processing methods. By performing such a measure, with regard to the material, completely homogeneous single component implantable catheters may be provided comprising at least one uninterrupted longitudinally oriented exchange area towards the tissue. Such a catheter may allow, after implantation, the continuous supply and/or drain of substances of any desired molecular size into the living tissue and/or out of the living tissue.

Such a method of manufacturing catheters with reduced risk of forming fluid tight occlusions may include the integration of filament shaped materials in the fluid guiding catheter lumen, wherein multi-fibril/medical sewing material may be used for this purpose.

When manufacturing such a catheter, the twisting of the remaining material (for instance PTFE) may generate a tissue supporting and highly flexible structure, wherein the character or properties of such a catheter may be stabilized by subsequent heating and cooling.

A method for connecting single or multi-luminal plastic catheters using implantation aids may include squeezing and inserting the material in a hollow needle after partial cross-sectional tapering using pulling forces and/or heat influence.

For implantation such a single or multi-luminal catheter in living tissue, a part of the catheter may be positioned in a hollow needle, whereas the remaining part of the catheter is returned or guided back along an exterior surface of the hollow needle.

It is possible to use a single luminal tube profile as a starting material, or alternatively a two- or more luminal tube material as a starting material.

It is possible to use such a catheter for a combined proof of physiologically active substances in an organ to be treated (for instance tissue) and possible effects of a local impact. Such samples may then be examined with respect to parameters indicative of an inflammation.

Next, further exemplary embodiments of the invention will be explained. In the following, further exemplary embodiments of the catheter will be explained. However, these embodiments also apply for the medical device, for the method of manufacturing a catheter, for the medical method and for the method of using a catheter for measuring a physiological parameter. The delivery unit may comprise a perfusion fluid container containing the perfusion fluid and being in fluid communication with the lumen of the structure. Such a perfusion fluid container may be a reservoir holding the perfusion fluid. The perfusion fluid container may contain a medication, particularly insulin. The insulin supply to the organism may be made dependent on the glucose concentration in the organism. The perfusion fluid may be used for both detecting the glucose concentration in the surrounding blood and for supplying a proper dose of insulin to control the glucose concentration to a desired value.

The catheter may comprise a drain unit for draining the perfusion fluid after the exchange of substances between the tissue and the perfusion fluid via the oblong slit. The drain unit may comprise a perfusion fluid collector collecting the perfusion fluid after the exchange of substances between the tissue and the perfusion fluid via the oblong slit. Such a collector may be a waste container or may be a member in which the perfusion fluid is analyzed after exchange with the body fluid. Such an analysis may include the measurement of a concentration of a substance.

The delivery unit and/or the drain unit may comprise a perfusion fluid transport unit, particularly a pump, for instance a peristaltic pump, for transporting the perfusion fluid through the lumen of the structure. Transport of the fluid may be carried out by pumping, sucking, etc. The catheter may be operated, for example, in a push mode, in a pull mode, or in a push-pull mode.

The drain unit may comprise an analysis unit adapted for analyzing the perfusion fluid after the exchange of substances between the tissue and the perfusion fluid via the oblong slit to thereby derive information regarding the tissue. Such an analysis may include the determination of the presence or absence of a substance, the determination of the concentration of a substance, and/or a calibration.

The delivery unit is be connected to a first end portion of the structure, and the drain unit is connected to a second end portion of the structure. Thus, the transport of the perfusion fluid may be effected in a first direction, whereas the exchange between the perfusion fluid and the surrounding organism may be effected in a second direction which may be essentially perpendicular to the first direction.

The catheter is adapted as a microperfusion and/or microdialysis catheter. In other words, substances may be exchanged via the slit(s) of the tubular wall in a similar manner as in the fields of microperfusion and/or microdialysis.

An interior of the tube may accommodate exactly one lumen. In such a scenario, the raw material for forming the catheter may be a tubular film which is simply treated with a simple scalpel. Such a manufacturing procedure is very simple.

Alternatively, an interior of the tube may accommodate a plurality of lumen. Even for such a catheter, standard materials are available with low cost. For instance, as a raw material, a tube having an essentially circular cross-section may be used, wherein along the circumference of the cross-section a plurality of cylindrical tubes are arranged adjacent to one another. Using such a raw material, a highly complex fluid communication system may be provided and even sophisticated fluid transfer applications may be carried out.

The catheter may comprise a plurality of oblong slits formed in the wall of the tube. Each of the plurality of oblong slits may be formed along the longitudinal axis of the catheter, that is to say along a symmetry axis of the tube. Alternatively, at least a part of the oblong slits may be formed along another direction, for instance circumferentially along a part of the circumference of the tube, or in a helical manner, in a zigzag manner, or along other desired trajectory. The selection of the shape of the oblong slits may allow to adjust the catheter for specific flexibility and mechanical support properties and requirements.

The catheter may be integrally formed. In other words, the catheter may be made from one piece of a single material. In such a configuration; the catheter can be manufactured with very low cost.

The catheter may be formed of exactly one material. For instance, a plastic material like PTFE or Teflon may be used. However, any other flexible biocompatible material may be used.

The catheter may consist of a single contiguous component. This may have the consequence that the catheter has a high degree of stability and, due to the formation of channels, simultaneously a high degree of flexibility. No adhering or other connection or attachment elements need to be provided which may simplify the catheter and may make the catheter particularly suitable for use within a living body.

The tube may be a cylindrical tube, for instance a cylindrical film. Therefore, the tube may form a hollow cylinder.

The tube may be twisted or may be twistable in a helical manner permanently or temporarily. For instance, the tube may be twisted only for insertion or implantation into the human body. Afterwards, the twisting may be removed or the degree of twisting reduced so as to open the channels again to enable fluid communication. Twisting may enable both, temporarily closing the fluid communication channel and reducing the diameter of the tube. This may be desired for inserting the tube into the body. It is also possible to remain the tube permanently in a twisted state, for instance when the tube comprises a shape memory material. With a shape memory material, the tube may be permanently held in a twisted state and, only when the temperature is raised above a threshold value, the material goes back to its original shape, for instance cylindrical shape. The required temperature may be supplied by the body temperature so that the tube may take its original shape automatically when being inserted into a living organism.

The oblong slit may have a dimension along the longitudinal axis of the tube which is significantly larger, particularly at least five times, more particularly at least ten times, preferably at least twenty times larger than a dimension along a circumference of the tube. Thus, essentially one-dimensional structures may be provided with a width which is essentially less than the length.

In the following, further exemplary embodiments of the method of manufacturing a catheter will be explained. However, these embodiments also apply for the catheter, the medical device, the medical method and the method of using a catheter.

The oblong slit may be formed in the wall of the tube using one of the group consisting of cutting, laser processing, blanking, drilling, and molding. In principle, cutting may be preferred, since this only requires a small scalpel for manufacturing the catheter in a few minutes or less.

The method may further comprise twisting the tube in a helical manner. By taking this measure, the catheter can be brought in an operation state, temporarily or permanently, in which it may be insertable in an easy manner into a human body. Furthermore, any fluid exchange may be made impossible in the twisted state, since the oblong slit is closed by the twisting procedure. After insertion into the body, the original shape of the tube may be restored.

The method may comprise treating the twisted tube to remain permanently in the twisted state by heating the twisted tube for a predetermined time interval and subsequently cooling the twisted tube. This procedure is easy and efficient for generating a permanently twisted tube. However, this twisted tube may be brought into its original shape, for instance by heating the tube above a threshold temperature.

A medical yarn or filament or a surgical sewing material may be inserted into the catheter. This may simplify use of the catheter, as explained above.

In the following, examples of the medical method will be explained. However, these examples also apply for the method of using such a catheter for estimating a physiological parameter.

During the medical method, an insertion needle may be connected to the catheter. Using such an insertion needle may simplify inserting the catheter into tissue of a human body.

Particularly, the insertion needle may be attached to the catheter by pulling the tube so as to reduce a diameter of the tube for inserting the tube with the reduced diameter in the insertion needle. This may facilitate connection of needle and catheter.

Additionally or alternatively, the insertion needle may be connected to the catheter by inserting a part of the tube into the insertion needle and by returning another part of the tube outside of the insertion needle. Inserting such a configuration into a human body and then slowly drawing back the needle may remove the needle from the catheter and may allow to maintain the catheter in the human body, but not the needle.

In the following, examples of the method of using a catheter for measuring a physiological parameter will be explained. However, these examples also apply for the medical method.

The catheter may be used for measuring a concentration of a physiologically active substance in tissue of a physiological object. By measuring the concentration of a physiologically active substance at a specific position within the body of the human being, the impact of an external influence, for instance contacting the body with a product like a cosmetics or a medication, can be investigated.

The method may further comprise using the catheter for measuring an effect of a physiologically active substance in tissue of a physiological object. Thus, not only the physiologically active substance itself (for instance insulin) may be measured, but also the impact thereof.

Furthermore, according to the method, the catheter may be used for determining an advantageous concentration of a physiologically active substance in tissue of a physiological object. In other words, the catheter may be used in the context of developing a new medication by optimizing a concentration of the medication to obtain a certain impact.

The catheter may further be used for determining a physiological parameter in tissue of a physiological object.

The aspects defined above and further aspects of the invention are apparent from the examples.of embodiment to be described hereinafter and are explained with reference to these examples of embodiment.

The invention will be described in more detail hereinafter with reference to examples of embodiment but to which the invention is not limited.
Fig. 1 shows a catheter according to an exemplary embodiment of the invention.
Fig. 2 shows a medical device according to an exemplary embodiment of the invention.
Fig. 3A to Fig. 3C show a medical device used for carrying out a medical method according to an exemplary embodiment of the invention.
Fig. 4 to Fig. 8 show catheters according to exemplary embodiments of the invention.
Fig. 9 shows results of a method of determining physiological parameters using catheters according to an exemplary embodiment of the invention.
Fig. 10 shows a catheter system according to an exemplary embodiment of the invention.

The illustration in the drawing is schematically. In different drawings, similar or identical elements are provided with the same reference signs.

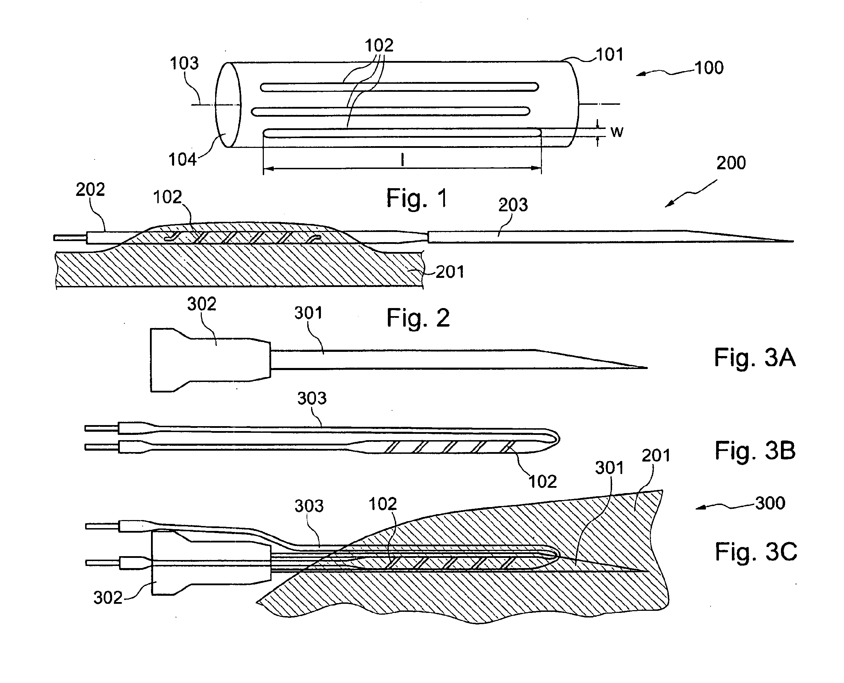

In the following, referring to **Fig. 1**, a catheter 100 according to an exemplary embodiment of the invention will be explained.

The catheter 100 is adapted for insertion into tissue of a human patient. The catheter 100 comprises a tube 101 made of Teflon material, which is a flexible biocompatible fluid-impermeable material. A plurality of oblong slits 102 are formed parallel to one another in a wall of the tube 101 and extend along a longitudinal axis 103, that is to say a symmetry axis, of the tube 101.

An interior 104 of the tube 101 accommodates exactly one lumen. A fluid flow or an exchange of substances is possible through the oblong holes 102. The catheter 100 is integrally formed from a single material, namely Teflon. The catheter 100 consists of a single contiguous component, namely the tube 101 in which the holes 102 have been cut using a scalpel. The length "I" of the oblong holes 102 is 20mm, and the width "w" is 0.2mm.

In the following, referring to **Fig. 2**, a medical device 200 according to an exemplary embodiment of the invention will be explained.

Fig. 2 shows the skin 201 of a living human organism in which the catheter 202 shall be inserted. Again, the catheter 202 consists of a single material, namely Polytetrafluoroethylene (PTFE) having an oblong hole 102 formed in a wall thereof. The catheter 202 has been helically wound which can be seen from the twisted shape of the oblong slit 102. Furthermore, for inserting the catheter 202 into an insertion needle 203, a pulling force has been applied to the catheter in longitudinal direction so as to taper a front end portion of the catheter 202 for simplified insertion into an opening at the end of the insertion needle 203. In this configuration, the array of catheter 202 inserted into the insertion needle 203 can be inserted into the tissue 201.

Therefore, Fig. 2 shows a linear embodiment of a sampling helix with an attached hollow needle for the application in surface tissue, for instance skin or subcutaneous fatty tissue. The insertion needle 203 has a diameter of 0.5 mm. The helical catheter 202 has a diameter of 0.5 mm (when the insertion needle 203 and the helical catheter 202 have the same diameter, e.g. 0.5 mm or 0.4 mm, this may be advantageous as tissue trauma may be reduced).

Fig. 3A to Fig. 3C show a medical device 300 according to another exemplary embodiment of the invention.

**Fig. 3A** again shows an insertion needle 301 including a grip 302 as a part (plastic) to make a connection to a syringe or to a tubing..

A catheter 303 shown in Fig. 3B is bent so as to form two essentially equally long portions.

As shown in **Fig. 3C**, half a portion of the catheter 303 is inserted into the needle 301 and the other portion is returned back outside of the needle 301. This arrangement can then, as shown in Fig. 3C, be implemented in tissue 201. Subsequent sufficiently slow removal of the insertion needle 301 may then keep the catheter 303 inside the tissue 201.

In other words, Fig. 3A to Fig. 3C show a sampling helix for the application in deeper tissue and the illustration of a possibility of introducing the sampling helix into the tissue 201.

The injection needle 301 may have a diameter of 0.8 mm. The diameter of the helical catheter 303 may be 0.4 mm.

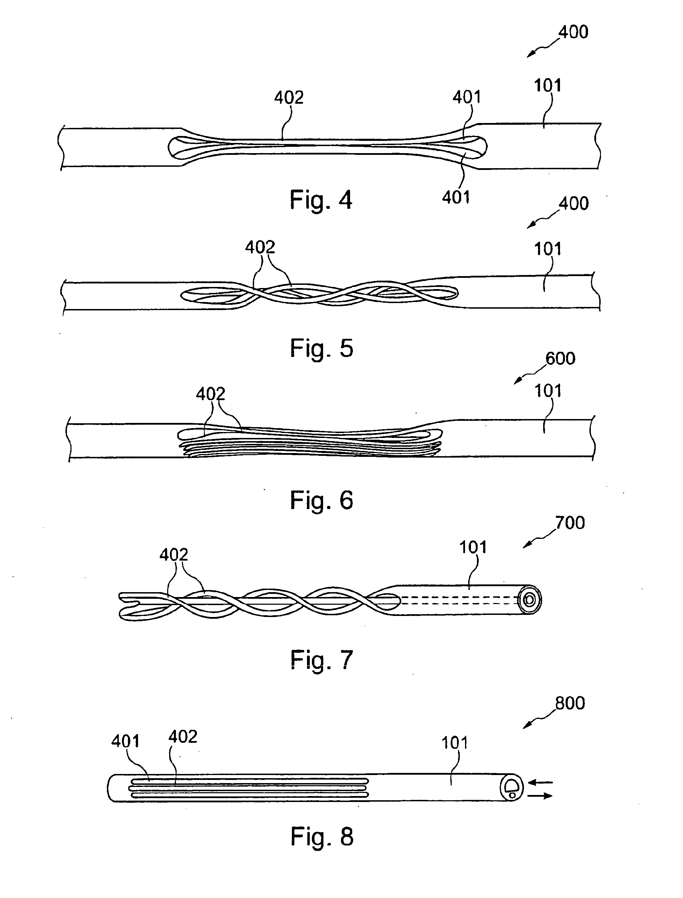

Next, referring to **Fig. 4**, a catheter 400 according to an exemplary embodiment of the invention will be explained.

Fig. 4 shows the catheter 400 which is made of a tubular material 101 in which a plurality of longitudinal cuts 401 have been formed so as to provide a plurality of connecting strips 402.

**Fig.** 5 shows again the linear catheter 400 of Fig. 4 in an operation state in which the two end portions have been twisted with respect to one another so that the filaments 402 form a twisted structures. The flexible structures 402 have been woven within one another.

**Fig. 6** shows another catheter 600 including a plurality of longitudinal cuts so that again a plurality of filaments 402 are generated.

**Fig. 7** shows a catheter 700 according to another exemplary embodiment of the invention.

The catheter 700 is contacted from a single side, namely the righthand side of Fig. 7. The catheter 700 is derived from the linear basic form as shown in Fig. 4. For example, the catheter 700 may be inserted into the tissue 201 using an insertion needle, like needle 203 or 301.

As can further be taken from Fig. 7, a double lumen catheter 700 is provided having an interior lumen for transporting fluid from the left side to the right side and an outer lumen for transporting fluid from the right side to the left side.

**Fig. 8** shows another catheter 800 according to an exemplary embodiment having two lumen, as indicated by the two arrows illustrating the fluid flow directions.

The catheter 800 is again a single side contactable catheter with a backflow, manufactured from a multi-lumen tube. The catheter 800 can be inserted into the tissue, for instance using a hollow needle.

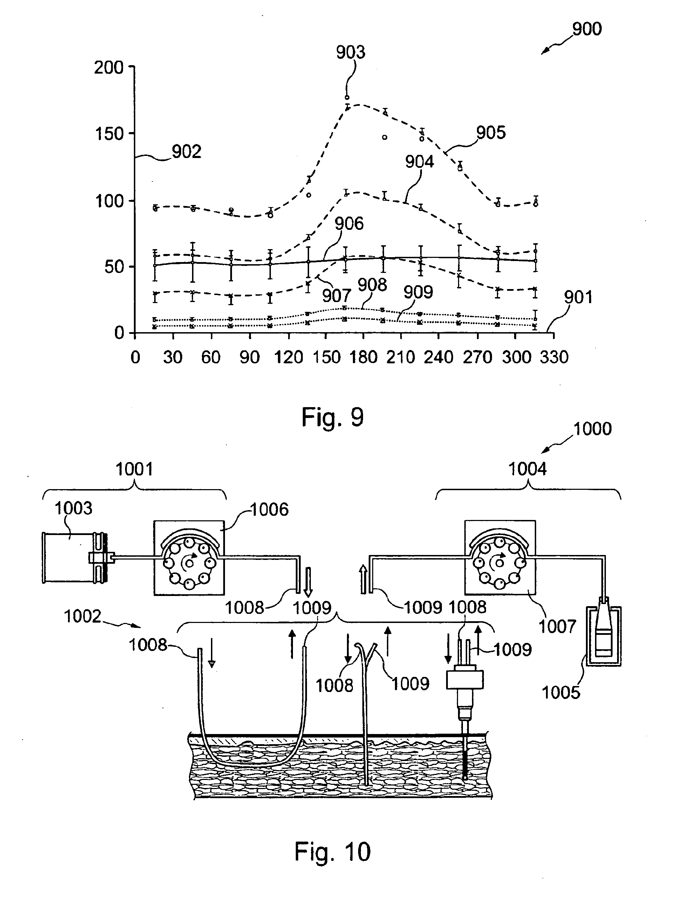

**Fig. 9** shows a diagram 900 illustrating an experiment performed using a catheter according to an exemplary embodiment.

Along an abscissa 901 of the diagram 900, the time is plotted in minutes. Along an ordinate 902 of the diagram 900, the glucose or lactate level in mg/dl is plotted, and the recovery in percent.

Circles 903 indicate a capillary glucose level, a curve 904 indicates an interstitial fluid glucose level. A curve 905 indicates a calibrated interstitial fluid glucose level. A curve 906 indicates a sodium recovery level. A curve 907 indicates a sample glucose. A curve 908 indicates an interstitial fluid lactate level. A curve 909 indicates a sample lactate 909.

Therefore, Fig. 9 illustrates the glucose and lactate concentration during a carbohydrates rich meal measured with four parallel arranged catheters in the human skin with a flow of 1µl/min. The sample concentrations of glucose and lactate have been calibrated by sodium (ionic reference technique) to obtain the interstitial fluid (ISF) concentration. The four interstitial fluid glucose profiles have been calibrated to capillary blood using a single point calibration at minute 15 to the capillary blood glucose (hand glucometer, single measurement). The low remaining lactate values indicate the proper suitability of the catheters in the human body. The error ranges indicate the standard deviation of the four measurements.

In the following, referring to **Fig. 10****, a** catheter system 1000 according to an exemplary embodiment of the invention will be explained.

The catheter system 1000 comprises a delivery unit 1001 for delivery of perfusion fluid to a lumen of a tubular catheter structure 1002 in a manner to allow for an exchange of substances between the tissue and the perfusion fluid via slits formed in the tubular catheter structure 1002 (not shown in Fig. 10).

The delivery unit 1001 comprises a perfusion fluid container 1003 containing the perfusion fluid and being in fluid communication with the tubular catheter structure 1002.

The catheter system 1000 further comprises a drain unit 1004 for draining the perfusion fluid after the exchange of substances between the tissue and the perfusion fluid via the oblong slits of the tubular catheter structure 1002. The drain unit 1004 comprises a perfusion fluid collector 1005 collecting the perfusion fluid after the exchange of substances between the tissue and the perfusion fluid via the oblong slit of the tubular catheter structure 1002.

The delivery unit 1001 comprises a first pump 1006 and the drain unit 1004 comprise a second pump 1007, both for transporting the perfusion fluid through the lumen of the tubular catheter structure 1002.

The delivery unit 1001 is connected to a first end portion 1008 of the tubular catheter structure 1002, and the drain unit 1004 is connected to a second end portion 1009 of the tubular catheter structure 1002.

Fig. 10 is a schematic representation of a system 1000 for the perfusion of tissue/an organism/a unit cell structure in connection with a catheter according to an exemplary embodiment of the invention. Three catheter designs 1002 are shown exemplarily. Catheters 1002 feature an exchange area towards the organism and two connections 1008, 1009 to a peripheral system 1001, 1004. System 1001, 1004 and catheter 1002 allow the simultaneous inflow of a perfusion fluid, and outflow of the perfusion fluid after interchange with the organism across the catheter's exchange area. The schematics of Fig. 10 shows two pumps 1006, 1007, here exemplarily peristaltic pumps. In principle any kind of pump or mechanism can be utilized that leads to a flow of fluid through the system 1000.

It should be noted that the term "comprising" does not exclude other elements or steps and the "a" or "an" does not exclude a plurality. Also elements described in association with different embodiments may be combined.

It should also be noted that reference signs in the claims shall not be construed as limiting the scope of the claims.

## Claims

1. A microperfusion catheter system comprising:
a microperfusion catheter (100, 200, 400, 600, 700, 800, 1002) for insertion into tissue of a physiological object, the catheter comprising:
a structure (101) made of a flexible biocompatible, impermeable material, the structure having a lumen, a first end portion and a second end portion;
an oblong slit (102) formed in a wall of the structure;
the microperfusion catheter system further comprising:
a delivery unit (1001) for delivery of perfusion fluid to the lumen (104) of the structure in a manner to allow for an exchange of substances between the tissue and the perfusion fluid via the oblong slit,
wherein the delivery unit is connected to the first end portion (1008) of the structure for delivering the perfusion fluid to the lumen of the structure, wherein a drain unit is connectable to the second end portion (1009) of the structure for draining the perfusion fluid after the exchange of substances between the tissue and the perfusion fluid via the oblong slit.

2. The microperfusion catheter system of claim 1,
wherein the delivery unit comprises a perfusion fluid container (1003) containing the perfusion fluid and being in fluid communication with the lumen of the structure, particularly comprises a perfusion fluid container containing a medication.

3. The microperfusion catheter system of any one of claims 1 to 2, comprising a drain unit (1004) for draining the perfusion fluid after the exchange of substances between the tissue and the perfusion fluid via the oblong slit.

4. The microperfusion catheter system of claim 3,
wherein the drain unit comprises a perfusion fluid collector (1005) collecting the perfusion fluid after the exchange of substances between the tissue and the perfusion fluid via the oblong slit.

5. The microperfusion catheter system of any one of claims 1 to 4,
wherein at least one of the delivery unit and the drain unit comprises a perfusion fluid transport unit (1006, 1007), particularly a pump, for transporting the perfusion fluid through the lumen of the structure.

6. The microperfusion catheter system of any one of claims 3 to 5,
wherein the drain unit comprises an analysis unit adapted for analyzing the perfusion fluid after the exchange of substances between the tissue and the perfusion fluid via the oblong slit to thereby derive information regarding the tissue.

7. The microperfusion catheter system of any one of claims 1 to 6, adapted as a microperfusion and/or microdialysis catheter.

8. The microperfusion catheter system of any one of claims 1 to 7, comprising a plurality of oblong slits (102, 402) formed in the wall of the structure.

9. The microperfusion catheter system of any one of claims 1 to 8,
wherein the structure (101) is a tube which is twisted or twistable in a helical manner permanently or temporarily.

10. A device, comprising
a microperfusion catheter system according to any one of claims 1 to 9;
an insertion element (203, 301) to be connected to the microperfusion catheter for facilitating insertion of the catheter into the tissue of the physiological object.

11. The device of claim 10,
wherein the insertion element is an insertion needle.

12. The device of claim 10 or 11,
wherein the insertion element comprises a first end portion adapted for receiving the microperfusion catheter and comprises a second end portion adapted for penetrating into the tissue.

13. The device of claim 12,
wherein the insertion element is adapted such that the microperfusion catheter is insertable into the first end portion, guidable through the insertion element, and leadable out of the insertion element through the second end portion.

14. A method of manufacturing a microperfusion catheter system comprising a microperfusion catheter (100, 200, 400, 600, 700, 800, 1002) for insertion into a physiological object, the method comprising
providing a structure (101) made of a flexible biocompatible, impermeable material, the structure having a lumen, a first end portion and a second end portion;
forming an oblong slit (102) in a wall of the structure;
forming a delivery unit (1001) for delivery of perfusion fluid to the lumen (104) of the structure in a manner to allow for an exchange of substances between the tissue and the perfusion fluid via the oblong slit, wherein the delivery unit is connected to the first end portion (1008) of the structure for delivering the perfusion fluid to the lumen of the structure, wherein a drain unit is connectable to the second end portion (1009) of the structure for draining the perfusion fluid after the exchange of substances between the tissue and the perfusion fluid via the oblong slit.

15. The method of claim 14,
comprising forming the oblong slit in the wall of the structure using at least one of the group consisting of cutting, laser processing, blanking, drilling, and molding.

16. The method of any one of claims 14 to 15,
comprising inserting medical yarn in the microperfusion catheter.

## Patentansprüche

1. Ein Mikroperfusionskathetersystem, welches aufweist:
einen Mikroperfusionskatheter (100, 200, 400, 600, 700, 800, 1002) zum Einführen in ein Gewebe eines physiologischen Objekts, wobei der Katheter aufweist:
eine Struktur (101), welche aus einem flexiblen biokompatiblen, impermeablen Material hergestellt ist, wobei die Struktur ein Lumen, einen ersten Endabschnitt und einen zweiten Endabschnitt hat,
einen länglichen Schlitz (102), welcher in einer Wand der Struktur gebildet ist,
wobei das Mikroperfusionskathetersystem ferner aufweist:
eine Zuführeinheit (1001) zum Zuführen eines Perfusionsfluids an das Lumen (104) der Struktur in einer Art und Weise, um einen Austausch von Substanzen über den länglichen Schlitz zwischen dem Gewebe und dem Perfusionsfluid zu erlauben,
wobei die Zuführeinheit mit dem ersten Endabschnitt (1008) der Struktur zum Zuführen des Perfusionsfluids an das Lumen der Struktur verbunden ist,
wobei eine Abflusseinheit mit dem zweiten Endabschnitt (1009) der Struktur zum Abfließen Lassen des Perfusionsfluids nach dem Austausch der Substanzen über den länglichen Schlitz zwischen dem Gewebe und dem Perfusionsfluid verbindbar ist.

2. Das Mikroperfusionskathetersystem gemäß Anspruch 1,
wobei die Zuführeinheit einen Perfusionsfluidcontainer (1003) aufweist, welcher das Perfusionsfluid enthält und in fluidischer Kommunikation mit dem Lumen der Struktur ist, insbesondere einen Perfusionsfluidcontainer aufweist, welcher ein Medikament enthält.

3. Das Mikroperfusionskathetersystem gemäß irgendeinem der Ansprüche 1 bis 2, welches eine Abflusseinheit (1004) zum Abfließen Lassen des Perfusionsfluids nach dem Austausch der Substanzen über den länglichen Schlitz zwischen dem Gewebe und dem Perfusionsfluid aufweist.

4. Das Mikroperfusionskathetersystem gemäß Anspruch 3,
wobei die Abflusseinheit einen Perfusionsfluidkollektor (1005) aufweist, welcher das Perfusionsfluid nach dem Austausch der Substanzen über den länglichen Schlitz zwischen dem Gewebe und dem Perfusionsfluid sammelt.

5. Das Mikroperfusionskathetersystem gemäß irgendeinem der Ansprüche 1 bis 4, wobei zumindest eine von der Zuführeinheit und der Abflusseinheit eine Perfusionsfluidtransporteinheit (1006, 1007) aufweist, insbesondere eine Pumpe, zum Transportieren des Perfusionsfluid durch das Lumen der Struktur hindurch.

6. Das Mikroperfusionskathetersystem gemäß irgendeinem der Ansprüche 3 bis 5,
wobei die Abflusseinheit eine Analyseeinheit aufweist, welche zum Analysieren des Perfusionsfluids nach dem Austausch der Substanzen über den länglichen Schlitz zwischen dem Gewebe und dem Perfusionsfluid angepasst ist, um dadurch Informationen abzuleiten, welche das Gewebe betreffen.

7. Das Mikroperfusionskathetersystem gemäß irgendeinem der Ansprüche 1 bis 6, welches als ein Mikroperfusions- und/oder Mikrodialysekatheter angepasst ist.

8. Das Mikroperfusionskathetersystem gemäß irgendeinem der Ansprüche 1 bis 7, welches eine Mehrzahl von länglichen Schlitzen (102, 402) aufweist, welche in der Wand der Struktur gebildet sind.

9. Das Mikroperfusionskathetersystem gemäß irgendeinem der Ansprüche 1 bis 8, wobei die Struktur (101) eine Röhre ist, welche permanent oder temporär in einer spiralförmigen Art und Weise verdrillt oder verdrillbar ist.

10. Eine Vorrichtung, welche aufweist
ein Mikroperfusionskathetersystem gemäß irgendeinem der Ansprüche 1 bis 9;
ein Einführelement (203, 301), welches an den Mikroperfusionskatheter zum Erleichtern eines Einführens des Katheters in das Gewebe des physiologischen Objekts anzuschließen ist.

11. Die Vorrichtung gemäß Anspruch 10, wobei das Einführelement eine Einführnadel ist.

12. Die Vorrichtung gemäß Anspruch 10 oder 11, wobei das Einführelement einen ersten Endabschnitt aufweist, welcher zum Empfangen des Mikroperfusionskatheters angepasst ist, und einen zweiten Endabschnitt aufweist, welcher zum Eindringen in das Gewebe angepasst ist.

13. Die Vorrichtung gemäß Anspruch 12,
wobei das Einführelement angepasst ist, so dass der
Mikroperfusionskatheter in den ersten Endabschnitt einsetzbar, durch das Einführelement hindurch führbar und aus dem Einführelement durch den zweiten Endabschnitt herausführbar ist.

14. Ein Verfahren zum Herstellen eines Mikroperfusionskathetersystems, welches einen Mikroperfusionskatheter (100, 200, 400, 600, 700, 800, 1002) zum Einführen in ein physiologisches Objekt aufweist, wobei das Verfahren aufweist
Bereitstellen einer Struktur (101), welche aus einem flexiblen biokompatiblen, impermeablen Material hergestellt ist, wobei die Struktur ein Lumen, einen ersten Endabschnitt und einen zweiten Endabschnitt hat;
Bilden eines länglichen Schlitzes (102) in einer Wand der Struktur,
Bilden einer Zuführeinheit (1001) zum Zuführen von Perfusionsfluid an das Lumen (104) der Struktur in einer Art und Weise, um einen Austausch von Substanzen über den länglichen Schlitz zwischen dem Gewebe und dem Perfusionsfluid zu erlauben,
wobei die Zuführeinheit mit dem ersten Endabschnitt (1008) der Struktur zum Zuführen des Perfusionsfluids an das Lumen der Struktur verbunden wird,
wobei eine Abflusseinheit mit dem zweiten Endabschnitt (1009) der Struktur zum Abfließen Lassen des Perfusionsfluids nach dem Austausch der Substanzen über den länglichen Schlitz zwischen dem Gewebe und dem Perfusionsfluid verbindbar ist.

15. Das Verfahren gemäß Anspruch 14, welches ein Bilden des länglichen Schlitzes in der Wand der Struktur mittels zumindest einem aus der Gruppe bestehend aus Schneiden, Laserverarbeiten, Stanzen, Bohren und Gießen aufweist.

16. Das Verfahren gemäß irgendeinem der Ansprüche 14 bis 15, welches ein Einführen von medizinischem Faden in den Mikroperfusionskatheter aufweist.

## Revendications

1. Système de cathéter de microperfusion comprenant :
un cathéter de microperfusion (100, 200, 400, 600, 700, 800, 1002) pour insertion dans du tissu d'un objet physiologique, le cathéter comprenant :
- une structure (101) constituée de matériau imperméable, biocompatible et flexible, la structure ayant une lumière, une première partie d'extrémité et une seconde partie d'extrémité ;
- une fente oblongue (102) formée dans une paroi de la structure ;
le système de cathéter de microperfusion comprenant en outre :
une unité d'alimentation (1001) pour alimenter en fluide de perfusion la lumière (104) de la structure de manière à permettre un échange de substances entre le tissu et le fluide de perfusion via la fente oblongue,
dans lequel l'unité d'alimentation est reliée à la première partie d'extrémité (1008) de la structure pour alimenter en fluide de perfusion à la lumière de la structure,
dans lequel une unité de drain peut être reliée à la seconde partie d'extrémité (1009) de la structure pour drainer le fluide de perfusion après l'échange de substances entre le tissu et le fluide de perfusion via la fente oblongue.

2. Système de cathéter de microperfusion selon la revendication 1, dans lequel l'unité d'alimentation comprend un contenant de fluide de perfusion (1003) contenant le fluide de perfusion et étant en communication fluidique avec la lumière de la structure, comprend en particulier un contenant de fluide de perfusion contenant une médication.

3. Système de cathéter de microperfusion selon l'une quelconque des revendications 1 à 2,
comprenant une unité de drain (1004) pour drainer le fluide de perfusion après l'échange de substances entre le tissu et le fluide de perfusion via la fente oblongue.

4. Système de cathéter de microperfusion selon la revendication 3, dans lequel l'unité de drain comprend un collecteur de fluide de perfusion (1005) collectant le fluide de perfusion après l'échange de substances entre le tissu et le fluide de perfusion via la fente oblongue.

5. Système de cathéter de microperfusion selon l'une quelconque des revendications 1 à 4,
dans lequel au moins l'une de l'unité d'alimentation et de l'unité de drain comprend une unité de transport de fluide de perfusion (1006, 1007), en particulier une pompe, pour transporter le fluide de perfusion à travers la lumière de la structure.

6. Système de cathéter de microperfusion selon l'une quelconque des revendications 3 à 5,
dans lequel l'unité de drain comprend une unité d'analyse adaptée pour analyser le fluide de perfusion après l'échange de substances entre le tissu et le fluide de perfusion via la fente oblongue pour en tirer des informations concernant le tissu.

7. Système de cathéter de microperfusion selon l'une quelconque des revendications 1 à 6,
adapté en tant que cathéter de microperfusion et/ou de microdialyse.

8. Système de cathéter de microperfusion selon l'une quelconque des revendications 1 à 7,
comprenant une pluralité de fentes oblongues (102, 402) formée dans la paroi de la structure.

9. Système de cathéter de microperfusion selon l'une quelconque des revendications 1 à 8,
dans lequel la structure (101) est un tube qui est enroulé ou enroulable de manière hélicoïdale de façon permanente ou temporaire.

10. Dispositif, comprenant,
un système de cathéter de microperfusion selon l'une quelconque des revendications 1 à 9 ;
un élément d'insertion (203, 301) à relier au cathéter de microperfusion pour faciliter l'insertion du cathéter dans le tissu de l'objet physiologique.

11. Dispositif selon la revendication 10,
dans lequel l'élément d'insertion est une aiguille d'insertion.

12. Dispositif selon la revendication 10 ou 11,
dans lequel l'élément d'insertion comprend une première portion d'extrémité adaptée pour recevoir le cathéter de microperfusion et comprend une seconde partie d'extrémité adaptée pour pénétrer dans le tissu.

13. Dispositif selon la revendication 12,
dans lequel l'élément d'insertion est adapté de telle sorte que le cathéter de microperfusion peut être inséré dans la première partie d'extrémité, guidé à travers l'élément d'insertion, et mené hors de l'élément d'insertion à travers la seconde partie d'extrémité.

14. Procédé de fabrication d'un système de cathéter de microperfusion comprenant un cathéter de microperfusion (100, 200, 400, 600, 700, 800, 1002) pour insertion dans un objet physiologique, le procédé comprenant
la fourniture d'une structure (101) constituée d'un matériau imperméable, biocompatible et flexible, la structure ayant une lumière, une première partie d'extrémité et une seconde partie d'extrémité ;
la formation d'une fente oblongue (102) dans une paroi de la structure ;
la formation d'une unité d'alimentation (1001) pour alimenter en fluide de perfusion la lumière (104) de la structure de manière à permettre un échange de substances entre le tissu et le fluide de perfusion via la fente oblongue, dans lequel l'unité d'alimentation est reliée à la première partie d'extrémité (1008) de la structure pour alimenter en fluide de perfusion la lumière de la structure, dans lequel une unité de drain peut être reliée à la seconde partie d'extrémité (1009) de la structure pour drainer le fluide de perfusion après l'échange de substances entre le tissu et le fluide de perfusion via la fente oblongue.

15. Procédé selon la revendication 14,
comprenant la formation de la fente oblongue dans la paroi de la structure à l'aide d'au moins une technique du groupe constitué par la coupe, le traitement laser, le découpage, le perçage et le moulage.

16. Procédé selon l'une quelconque des revendications 14 à 15, comprenant l'insertion d'un fil médical dans le cathéter de microperfusion.
